Europäisches Patentamt

European Patent Office

Office européen des brevets

(11) Publication number : **0 400 480 B1**

# EUROPEAN PATENT SPECIFICATION

(45) Date of publication of patent specification :
25.08.93 Bulletin 93/34

(51) Int. Cl.⁵ : **A61K 41/00**

(21) Application number : **90109869.9**

(22) Date of filing : **23.05.90**

(54) **A method for enhancing the cytotoxicity of anthracyclines through photoactivation.**

(30) Priority : **29.05.89 IT 2067989**

(43) Date of publication of application :
**05.12.90 Bulletin 90/49**

(45) Publication of the grant of the patent :
**25.08.93 Bulletin 93/34**

(84) Designated Contracting States :
**AT BE CH DE DK ES FR GB GR IT LI LU NL SE**

(56) References cited :
**BIOCHIMICA ET BIOPHYSICA ACTA, vol. 1014, 1989, Amsterdam (NL); A. ANDREONI et al., pp. 8-13**
**PHOTOCHEMISTRY PHOTOBIOLOGY, vol. 36, 1982, GB; P.J. GREY et al., pp. 49-57**

(73) Proprietor : **CONSIGLIO NAZIONALE DELLE RICERCHE**
**Piazzale Aldo Moro, 7**
**I-00198 Roma (IT)**

(72) Inventor : **Andreoni, Alessandra Maria**
**Via Lucullo, 132**
**I-80070 Bacoli, Napoli (IT)**
Inventor : **Colasanti, Alberto**
**Via Domenico Fontana, 135**
**I-80131 Napoli (IT)**
Inventor : **Malatesta, Vincenzo**
**Via della Repubblica, 21**
**I-20090 San Maurizio Al Lambro, Milan (IT)**
Inventor : **Roberti, Giuseppe**
**Via Porpora, 7/B**
**I-80128 Napoli (IT)**

(74) Representative : **Gervasi, Gemma, Dr. et al**
**NOTARBARTOLO & GERVASI Srl 33, Viale Bianca Maria**
**I-20122 Milano (IT)**

## Description

The present invention refers to a method for the in vitro photoactivation of daunorubicin or doxorubicin derivatives with the aid of light of wave length of between 500 and 700 nm, by means of which the cytotoxic activity of these substances is increased by a 10 to 10000 factor.

A particular interesting feature of the present invention is the use of radiations of wave lengths comprised within the red range of the visible spectrum.

The characteristics and advantages of the anthracyclines photoactivation method according to the present invention will be shown with more detail hereinafter.

Aliquots of a concentrated anthracycline solution in bidistilled deionized water are suitably diluted with a normal cell culture medium to obtain a concentration suitable for the incubation.

The cell cultures are prepared by routine methods both as far the culture medium and the container (e.g. a Petri dish, a flask etc.) are concerned.

To determine and to evaluate the cytotoxic effect, suitable routine techniques are employed selected according to the cell type, the effect which is to be determined (effect on cell growth, on the synthesis activity of the various cell components; morphologic, structural and ultrastructural effects, cell vitality in terms of percentage of vital cells, etc.).

The capsules are incubated with a fresh mediun containing the anthracycline for a time of between 1 and 48 hours.

The anthracycline concentration is normally below the value which gives the effect under study in a detectable way when the administration is made in the absence of radiations.

During the incubation the culture is irradiated with light of a wave length selected in the 500-700 nm range, within the visible absorption band of the anthracycline employed for the incubation, with an intensity of 5 to 500 mW/cm$^2$ and with a light dose of between 10 and 500 J/cm$^2$.

The light is administered in a direction perpendicular to the biological specimen and is produced by laser or by a suitably filtered lamp in order to obtain a radiation of the desired wave length.

The use of laser is essential when optical fibres or optical guidance techniques are used for the light transport.

The treatment is performed in a chamber in which optimum atmosphere and temperature conditions for the cell specimen under treatment are assured.

The irradiation is performed in the presence of the anthracycline and has a duration of between 15 min. and the total permanence time of the anthracycline in the cells.

We have proved that the irradiation during the first 30 min. of incubation with anthracycline is of relatively small effectiveness and that an optimum time of between 60 and 90 minutes exists to obtain the maximum immediate cytotoxic effect by photoactivation by means of a given light dose.

Anthracyclines suitable for the treatment are the daunorubicin (daunomicin) and doxorubicin (adriamicin) derivatives with substitutions which involve a shift of the visible absorption peak, which for the original daunomicin and adriamicin molecules is at about 480 nm, to wave lengths of between 500 and 700 nm. As examples, imino- and aminoderivatives are cited.

We have found that by the photoactivation treatment according to the invention, the cytotoxic activity, obtained with anthracycline doses which in the absence of photoactivation give only modest results, is increased by a factor of 10 when measured on the long term effect such as the cell growth in the days following the treatment, and by a factor of typically 10000 when measured on the immediate effect such as the cell death at the end of the treatment.

These results are extremely important in the oncologic field, in which, as known, the anthracyclines present cytotoxic activity, accompanied, however, by serious side effects. In fact, by the treatment according to the present invention it is possible to obtain high cytotoxic effects with reduced anthracycline dosage, thus reducing the side effects.

It is furthermore very important that the photoactivation takes place with the aid of a light of a wave length to which both tissues and blood are relatively transparent.

The following examples are reported for illustrative, not limitative, purposes.

<u>List of drawings</u>

The accompaying drawings are referring to:
- Figure 1 is a graphic representation of the count of vital cells pro ml as a function of the days following the insemination of dishes;
- Figure 2 is a graphic representation of the percentage of cell survival referred to a non-irradiated culture as a function of the light dose in $J/cm^2$;

- Figure 3 is graphic representation of the percentage cell survival, with respect to non-irradiated cultures, as a function of the delay from irradiation start with respect to the start of the incubation with 5-ID.

## Example 1

A culture of Fisher Rat Thyroid cells classified as FRT L5 was performed routinely in Petri dishes in a Ham F-12 medium modified by Coon (K.C. Biological Co.) with addition of 5% calf serum (Grand Island Biological Co.) by incubation at 37°C in an atmosphere with 5% $CO_2$.

For determining the growth curve a suitable number of the culture Petri dishes was trypsinized and the cells were re-suspended in growth medium in such a way as to have a cell concentration of appoximately $10^6$/ml. Suspension volumes containing a number of cells of the order of $10^5$ were poured into dishes of 60 mm diameter and covered with 3 ml culture medium, maintaining the cell suspension under stirring in order to have a uniform cell distribution in the various dishes.

The dishes were put in an incubator for 24 hrs and then incubated for 2 hrs with fresh reaction medium containing 5-iminodaunomicin (5-ID).

5-iminodaunomicin was prepared in a $1.78.10^{-5}$M solution in deionized bidistilled water and was added to the various dishes in such amounts as to have concentrations of 0,50, 200, 400 and 1000 ng/ml. Incubation of these dishes was performed in the dark while at the same time a dish prepared in the same way and having a 5-iminodaunomicin concentration of 50 ng/ml was incubated under red light radiation of 595 nm wave length.

The light reached the dish at a 90° angle and the transverse ray cross-section was higher than the dish section. The irradiation density was of 40 $mW/cm^2$ at the dish centre and 15 $mW/cm^2$ at the edge. The administered light dose was calculated on the basis of a 34 $mW/cm^2$ average density and was found to be 245 $J/cm^2$.

The culture was irradiated in a chamber in air flow at 37°C.

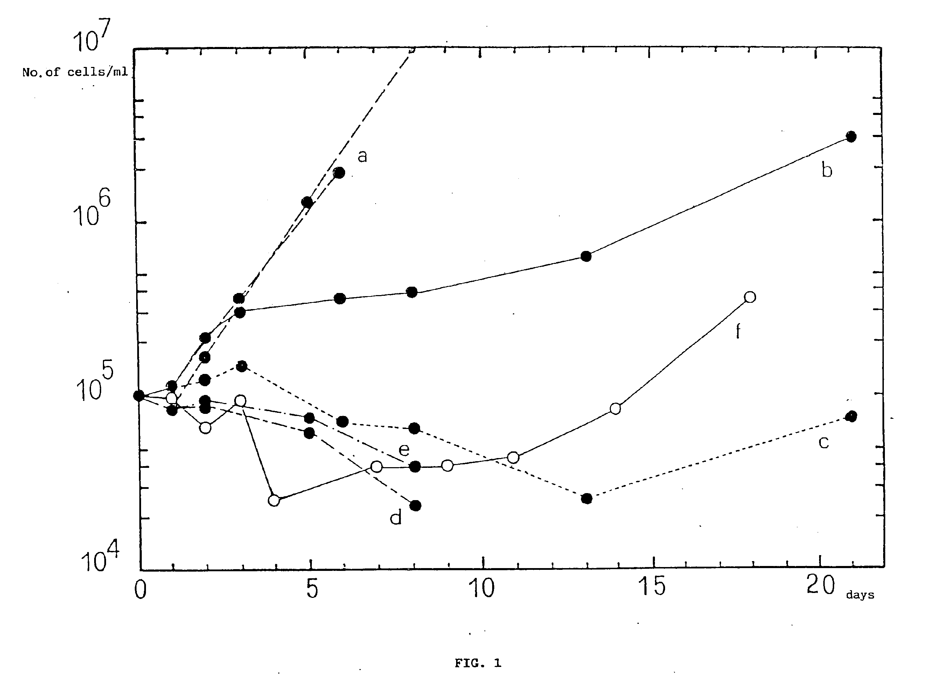

The results of the treatment are reported in fig. 1 in which the count of vital cells pro ml as a function of the days following the insemination of the dishes (day 0) and the 2 hr incubation (day 1). The meaning of the curves of fig. 1 is:

| Curve | Concentration 5-ID (ng/ml) | without irradiation | with irradiation (245 $J/cm^2$) |
|-------|----------------------------|---------------------|---------------------------------|
| (a) | 0 | x | |
| (b) | 50 | x | |
| (c) | 200 | x | |
| (d) | 400 | x | |
| (e) | 1000 | x | |
| (f) | 50 | | x |

Comparing curve (f) with curve (b) one sees how irradiation for two hours incubation with 50 ng/ml 5-ID results in lowering the number of vital cell of one order of magnitude.

## Example 2

Following the method of Example 1, FRT L5 cells were resuspended, distributed in dishes and incubated for 72 hrs. At this point 5-ID was introduced in the dishes in an amount such as to have a concentration in the medium of 1000 ng/ml and the dishes were incubated for 2 hrs. During this period the various dishes were irradiated with red light of 595 nm with constant radiation density on the dish of an average of 34 $mW/cm^2$ for times of from 15 to 120 minutes.

Each irradiation period was selected so that its termination coincided with the incubation end.

The average dose of light administered on the dish in 120 minutes was 245 $J/cm^2$.

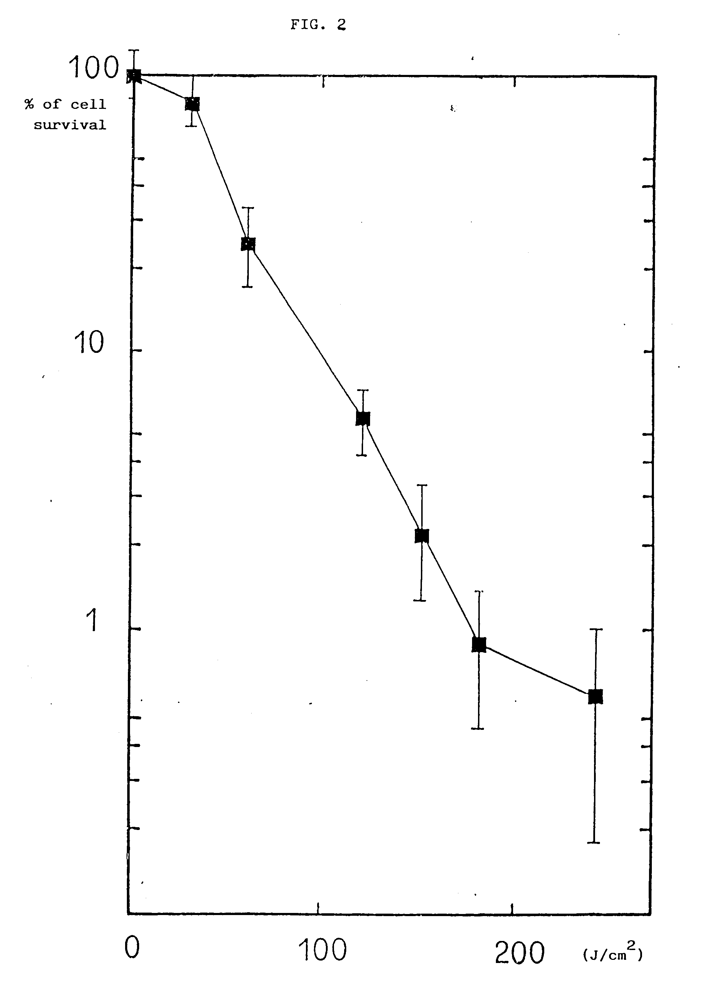

The results are reported in fig. 2, in which the percentage of cell survival refferred to a non-irradiated culture is represented as a function of the light dose in J/cm$^2$.

These results prove that the treatment of this example has a high lethal effect in a short time (ca. 30 minutes) while incubation without irradiation at the same 5-ID concentration in a short time does not have a measurable effect (see curve (e) of figure 1).

Example 3

Example 2 was repeated, except that each dish was irradiated for only 15 minute during the 2 hr incubation with 5-ID.

Said 15 minute irradiations were started with increasing delays with respect to the start of the incubation, and precisely with delays of 0, 15, 30, 45, 60, 75, 90 and 105 minutes.

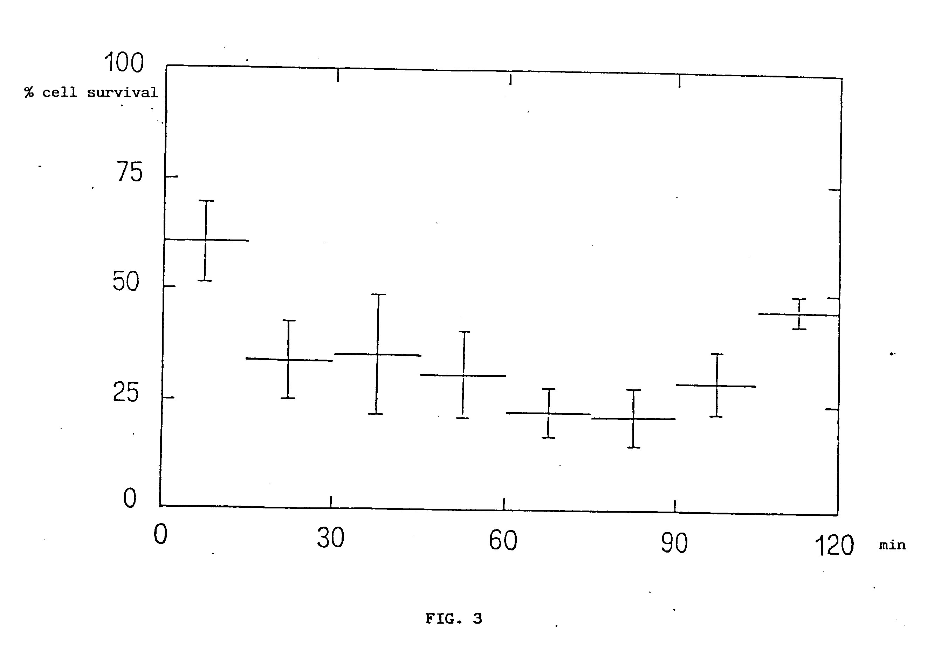

The results are reported in figure 3, in which the percentage cell survival, with respect to non- irradiated cultures, is reported as a function of the delay from the irradiation start with respect to the start of the incubation with 5-ID.

From this figure one can see how the best results are obtained with a delay of between 60 and 90 minutes.

## Claims

1. A method for enhancing the in vitro cytotoxicity characterized in that, during the permanence in the cells of derivatives of daunorubicin (daunomycin) and of doxorubicin (adriamycin) with substituents involving a shift of the visible absorption peak to wave lengths between 500 and 700 nm, a photoactivation process by irradiation with light of a wave length comprised between 500 and 700 nm is performed.

2. A method according to claim 1, characterized in that said irradiation is performed with an intensity of 5 to 500 mW/cm$^2$.

3. A method according to claim 1, characterized in that said irradiation is performed with a light dose of between 10 and 500 J/cm$^2$.

4. A method according to claim 1, characterized in that said irradiation is performed by means of laser light.

5. A method according to claim 1, characterized in that said irradiation is performed by means of light produced by lamps and suitably filtered.

6. A method according to claim 1, characterized in that the light is administered through guided light techniques.

7. A method according to claim 1, characterized in that said derivatives of daunomycin and adriamycin are the imino derivatives and the aminoderivatives.

8. A method according to claim 1, characterized in that said irradiation starts between 60 and 90 minutes after starting the incubation with said derivatives.

## Patentansprüche

1. Verfahren zur Erhöhung der in-vitro-Cytotoxizität, dadurch **gekennzeichnet,** daß während des Aufenthalts von Derivaten von Daunorubicin (Daunomycin) und von Doxorubicin (Adriamycin) mit Substituenten, die eine Verschiebung des Absorptionspeaks im sichtbaren Bereich zu Wellenlängen zwischen 500 und 700 nm bewirken, in den Zellen ein Photoaktivierungsprozeß durch Bestrahlung mit Licht einer Wellenlänge aus dem Bereich von zwischen 500 und 700 nm durchgeführt wird.

2. Verfahren gemäß Anspruch 1, dadurch **gekennzeichnet**, daß diese Bestrahlung mit einer Intensität von 5 bis 500 mW/cm$^2$ durchgeführt wird.

3. Verfahren gemäß Anspruch 1, dadurch **gekennzeichnet**, daß die Bestrahlung mit einer Lichtdosis von zwischen 10 und 500 J/cm$^2$ durchgeführt wird.

4. Verfahren gemäß Anspruch 1, dadurch **gekennzeichnet**, daß die Bestrahlung mit Laserlicht ausgeführt wird.

5. Verfahren gemäß Anspruch 1, dadurch **gekennzeichnet**, daß die Bestrahlung mittels Licht durchgeführt wird, das von Lampen erzeugt und geeignet gefiltert wird.

6. Verfahren gemäß Anspruch 1, dadurch **gekennzeichnet**, daß das Licht durch Lichtleitertechniken verabreicht wird.

7. Verfahren gemäß Anspruch 1, dadurch **gekennzeichnet**, daß die Daunomycin- und Adriamycinderivate die Iminoderivate und die Aminoderivate sind.

8. Verfahren gemäß Anspruch 1, dadurch **gekennzeichnet**, daß die Bestrahlung zwischen 60 und 90 Minuten nach Beginn der Inkubation mit diesen Derivaten beginnt.


## Revendications

1. Une méthode pour améliorer la cytotoxicité in vitro caractérisée en ce que, lors de la permanence dans les cellules de dérivés de daunorubicine (daunomicine) et de doxorubicine (adriamycine) ayant des substituants entraînant une variation dans le pic d'absorption visible à des longueurs d'ondes comprises entre 500 et 700 nm, on réalise un procédé de photo-activation par irradiation avec une lumière ayant une longueur d'onde comprise entre 500 et 700 nm.

2. Une méthode selon la revendication 1, caractérisée en ce que ladite irradiation est réalisée avec une intensité de 5 à 500 mW/cm$^2$.

3. Une méthode selon la revendication 1, caractérisée en ce que ladite irradiation est réalisée avec une dose de lumière comprise entre 10 et 500 J/cm$^2$.

4. Une méthode selon la revendication 1, caractérisée en ce que ladite irradiation est réalisée au moyen d'une lumière provenant d'un laser.

5. Une méthode selon la revendication 1, caractérisée en ce que ladite irradiation est réalisée au moyen d'une lumière produite par des lampes et convenablement filtrée.

6. Une méthode selon la revendication 1, caractérisée en ce que la lumière est administrée par des techniques de guidage de lumière.

7. Une méthode selon la revendication 1, caractérisée en ce que lesdits dérivés de daunomycine et d'adriamycine sont des dérivés imino et amino.

8. Une méthode selon la revendication 1, caractérisée en ce que le début de l'irradiation commence entre 60 et 90 minutes après l'incubation avec ces dits dérivés.

FIG. 1

FIG. 2

FIG. 3

EP 0 400 480 B1